# EUROPÄISCHE PATENTSCHRIFT

(11) **EP 1 245 219 B1**
(45) Veröffentlichungstag und Bekanntmachung des Hinweises auf die Patenterteilung: **07.09.2005**
(21) Anmeldenummer: 02005841.8
(22) Anmeldetag: 14.03.2002
(51) Int. Cl.: A61K 6/083, C08F 2/50

(54) **Aushärtbare Dentalzusammensetzung sowie Dentalsystem**
Curable dental composition and dental system
Composition dentaire durcissable et sytème dentaire

(30) Priorität: 26.03.2001 DE 10115001
(43) Veröffentlichungstag der Anmeldung: 02.10.2002
(73) Patentinhaber: Heraeus Kulzer GmbH, 63450 Hanau (DE)
(72) Erfinder: Schuh, Heinz, Dr., 40670 Meerbusch (DE); Erdrich, Albert, Dr., 61231 Bad Nauheim (DE); Greczmiel, Michael, Dr., 61273 Wehrheim (DE); Hoffmann, Dr. Marcus, 61250 Usingen (DE)
(74) Vertreter: Kühn, Hans-Christian

(56) Entgegenhaltungen:
- EP-A- 0 880 945
- US-B1- 6 204 302
- STAHL F ET AL: "Light-emitting diode (LED) polymerisation of dental composites: flexural properties and polymerisation potential" BIOMATERIALS, ELSEVIER SCIENCE PUBLISHERS BV., BARKING, GB, Bd. 21, Nr. 13, Juli 2000 (2000-07), Seiten 1379-1385, XP004197555 ISSN: 0142-9612
- WHITTERS C J ET AL: "CURING OF DENTAL COMPOSITES BY USE OF INGAN LIGHT-EMITTING DIODES" OPTICS LETTERS, OPTICAL SOCIETY OF AMERICA, WASHINGTON, US, Bd. 24, Nr. 1, 1999, Seiten 67-68, XP000801373 ISSN: 0146-9592

## Beschreibung

Die Erfindung betrifft ein Verfahren zum Aushärten einer (meth)acrylathaltigen Dentalzusammensetzung sowie ein Dentalsystem zur Durchführung des Verfahrens. Unter (meth)acrylathaltig sind Zusammensetzungen zu verstehen, die Methacrylate, Acrylate und/oder Gemische beider enthalten.

Im allgemeinen werden auf dem Gebiet der Dentaltechnik aushärtbare Dentalzusammensetzungen verwendet, die Monomere und Photoinitiatorsysteme enthalten, wobei diese in der Regel mit Halogenlampen aushärtend bestrahlt werden. Die üblicherweise 20 bis 60 Sekunden andauernde Aushärtungszeit lässt sich nicht beliebig durch eine Intensitätserhöhung der Halogenlampen beschleunigen, da eine Intensitätserhöhung auch zu erhöhten Emissionen im nahen UV-Bereich und zu einer erhöhten Wärmeleistung führt. Dies kann u.a. zur Versprödung der Dentalmaterialien führen bzw. können durch die erhöhte Wärmebelastung die Dentalmaterialien, aber vor allem das lebende Zahngewebe geschädigt werden. Somit wird dem Patienten eine relativ langwierige und eher als unangenehm empfundene Bearbeitungszeit aufgrund der Belichtungsdauer der Dentalmaterialien zugemutet.

Aus dem Vorgenannten ergibt sich das Problem, die Aushärtungszeit soweit wie möglich zu reduzieren und gleichzeitig die Wärmebelastung so gering wie möglich zu halten.

Dieses Problem wird erfindungsgemäß durch ein Verfahren zum Aushärten von (meth)acrylathaltigen Dentalzusammensetzungen mit einem bestimmten Photoinitiatorsystem und blauem LED-Licht der Wellenlänge 420 - 520 nm nach Anspruch 1 gelöst.

Bei dem erfindungsgemäßen Verfahren wird als Photoinitiatorsystem Campherchinon und/oder Phenanthrenchinon und 1-Phenyl-1,2-propandion und/oder ein ähnliches Diketon, kombiniert mit einem reduzierenden Amin, verwendet. Solche Initiatorsysteme wurden für Dentalmassen in US 6,204,302 vorgeschlagen. Auch eine Kombination der genannten Initiatoren mit Phosphinoxiden ist denkbar. Überraschenderweise eignen sich solche Initiatorsysteme für eine schnellere Härtung mit blauem LED-Licht.

Das Photoinitiatorsystem weist insbesondere im Wellenlängenbereich von 420 - 480 nm eine spezifische Absorption auf. Durch den im Verhältnis zur Halogenlampen-Emission schmaleren spezifischen Wellenlängenbereich ist es möglich, mit Hilfe der LED-Lichtquelle, die ein Emissionsmaximum um 468 nm hat, bei entsprechender hoher Intensität eine schnelle Aushärtung der gezielt für diese Wellenlänge kombinierten Dentalzusammensetzung bei gleichzeitig geringem Wärmeeintrag durchzuführen.

Die nachfolgenden Ausgestaltungen haben sich in der Praxis in vorteilhafter Weise bewährt.

Es handelt sich beim Photoinitiatorsystem um ein Campherchinon und/oder Phenanthrenchinon und 1-Phenyl-1,2-Propandion und/oder ein ähnliches Diketon und/oder ein Phosphinoxid (z. B. Lucirin TPO) kombiniert mit einem reduzierenden Amin.

Insbesondere handelt es sich beim reduzierenden Amin um eine Verbindung aus der Gruppe der tertiären aromatischen Amine.

Beim photopolymerisierbaren Monomer handelt es um ethylenisch ungesättigte Verbindungen aus der Gruppe der (Meth)acrylate. Bevorzugt sind Monomere aus der Gruppe Urethandi-(meth)acrylat (1), Bisphenol-A-Propyloxy-Di(meth)acrylat (2), Bisphenol-A-Glycidyl-Di(meth)-acrylat (3), Esther-Urethan-(meth)acrylsäurederivate eines Tricyclo-[5.2.1.0]-Decanderivates (4) und der cyclischen Carbonatacrylat/methacrylat-Verbindungen.

Für die Anwendung als Monomere können diese mit an sich bekannten niedermolekularen Monomeren gemischt werden, um beispielsweise die Viskosität dem Verwendungszweck anzupassen. Dies wird mit dem Zumischen von sogenannten Reaktivverdünnern erreicht. Insbesondere haben sich bewährt:
Aliphatische Di(meth)acrylate (5) wie z. B. Hexandiol-dimethacrylat, Ethylenglycoldi(meth)-acrylate und Polyethylenglycol-di(meth)acrylate (6) wie z.B. Triethylenglycoldimethacrylat und Hydroxyethyl(meth)acrylat (7).

Zur Verbesserung der Vernetzungsdichte, die eine Verbesserung der mechanischen und abrasionsresistenten Eigenschaften ermöglicht, können zu den beschriebenen Monomergemischen auch hochreaktive Mehrfachvernetzer aus der Gruppe der (Meth)acrylate beigemischt werden. Zum Einsatz kommen in vorteilhafter Weise insbesondere alkoxyliertes Penthaerythritol-Tetra(meth)acrylat (8) und 1,1,1-Trimethylol-propantri(meth)acrylat (9). Nachfolgend sind die genannten Verbindungen dargestellt.

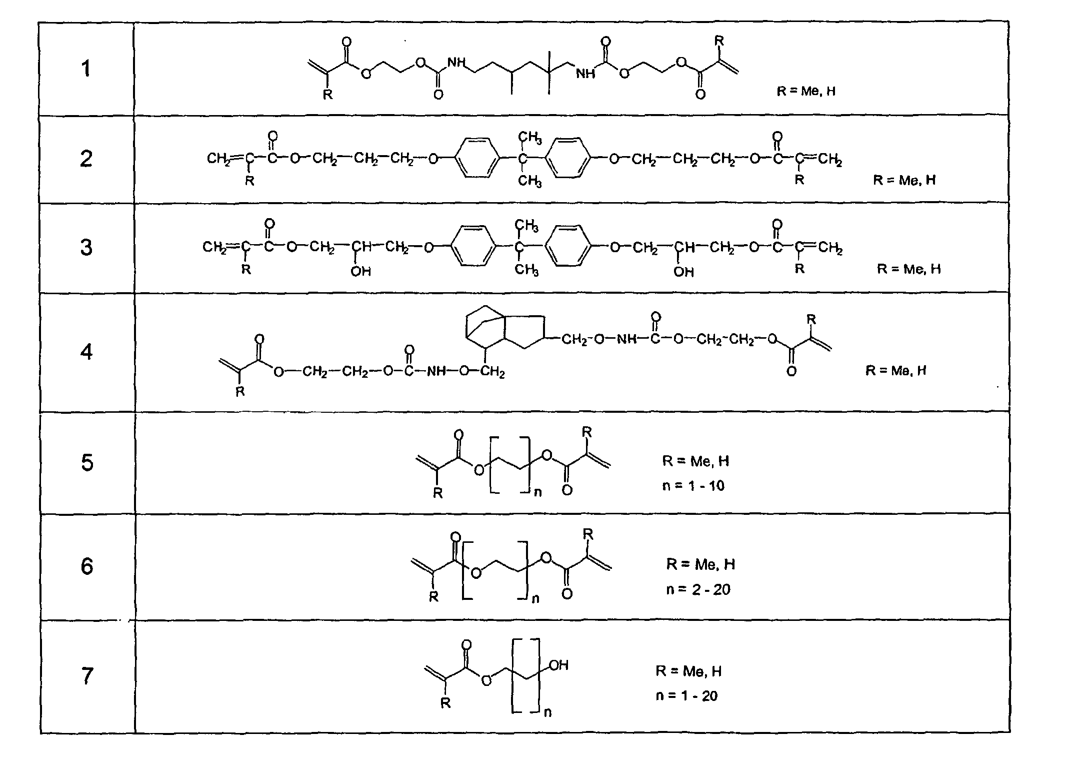

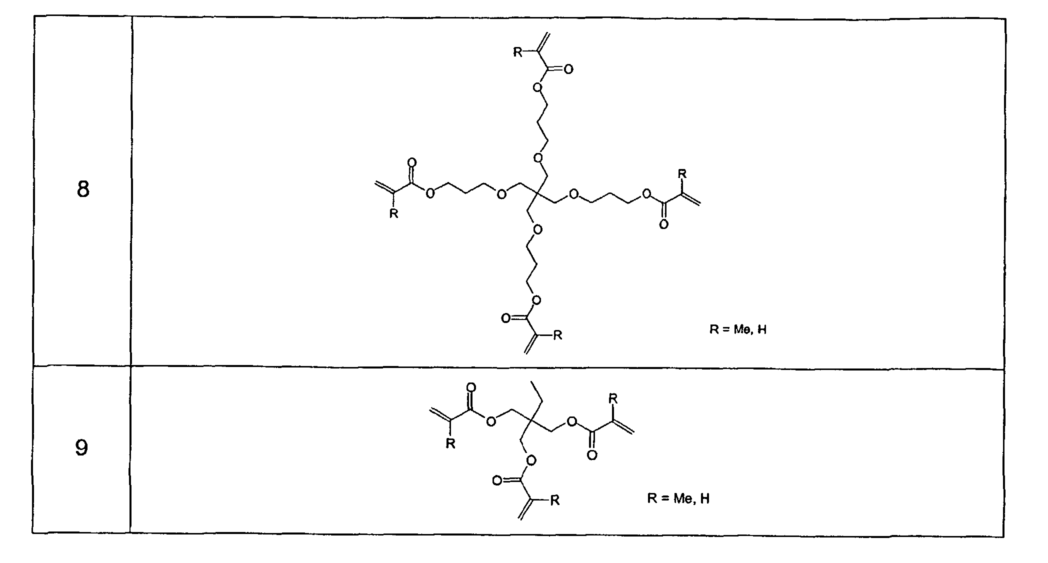

Bei der Auswahl der Monomeren kann es vorteilhaft sein, besonders reaktive und schnell härtende Monomere zu verwenden. Bewährt hat sich dabei ein größerer Anteil an Acrylatmonomeren im Gemisch mit Methacrylaten und/oder anderen ethylenisch ungesättigten Verbindungen.

Erfindungsgemäß geeignet als spezifische Lichtquelle ist ein LED-gespeistes Lichtgerät mit einem Emissionsspektrum im Bereich von 420 - 520 nm mit einem Emissionsmaximum bei 468 ± 10 nm. In diesem Wellenlängenbereich wird bei idealer Gauß'scher Verteilung mehr als 90 % der Energie emittiert. Das LED-Gerät kann auch nach dem Laserprinzip betrieben werden.

Überraschenderweise hat sich gezeigt, dass es möglich ist, durch Anpassung des Photoinitiatorensystems einer Dentalzusammensetzung an das Emissionsspektrum eines LED-Lichtgerätes bzw. durch eine gegenseitige Abstimmung eine wesentliche Verkürzung der für die vollständige Aushärtung der Dentalzusammensetzung nötigen Belichtungszeit (etwa eine Halbierung) gegenüber herkömmlichen Polymerisationszeiten zu erreichen. Diese Wirkung konnte bei der Aushärtung einer Dentalzusammensetzung ohne erfindungsgemäße Photoinitiatorenkombination weder durch Einsatz eines LED-Lichtgerätes allein noch durch Intensitätssteigerung von Halogenlampen erzielt werden.

Bei der erfindungsgemäß verwendeten Dentalzusammensetzung können als Füllstoffe feinstgemahlene dentale Gläser (Bariumaluminiumborsilicatglas, Bariumaluminiumborfluorsilicatglas, Strontiumaluminiumborsilicatglas (jeweils mit d₅₀ = 0,3 - 1,0 µm und d₉₉ = 3 - 5 µm)) eingesetzt werden, um hochglanzpolierbare röntgenopake Dentalkomposite zu erhalten. (Der Durchmesser von 50 % (d₅₀) bzw. 99 % (d₉₉) aller Teilchen ist hierbei kleiner als der angegebene Durchmesser in µm).

Quarz- oder Silikatglasfasern mit einem Durchmesser ≤ 0,1 - 0,5 µm und einer Länge von 1 - 100 µm sind vorteilhaft einzusetzen, um mechanisch sehr feste und bruchzähe Dentalkomposite zu erhalten.

Auch feinstgemahlene Keramikpulver mit d₅₀ = 0,3 - 1,0 µm und d₉₉ = 3 - 5 µm sind vorteilhaft einsetzbar, um mit keramischen Pulvern verstärkte Dentalkomposite zu erhalten.

Prepolymerpulver (gefüllt, ungefüllt, splittrig gemahlen oder kugelförmig, synthetisiert) ergeben hochglanzpolierbare dentale Verblendkomposite.

Mikrofüller (hochdisperses, gefälltes oder pyrolytisch gewonnenes SiO₂) werden vorteilhafter Weise zur Steuerung der rheologischen Eigenschaften, zur Erzielung opaleszierender optischer Eigenschaften und für die Herstellung hochglanzpolierbarer Dentalkomposite eingesetzt.

Radioopake Verbindungen wie beispielsweise schwerlösliche weiße Salze von Bariumsulfat, Strontiumfluorid, Yttriumfluorid und Cer- oder Lanthanoxiden können zur Herstellung von radioopaken Produkten eingesetzt werden.

Fluorid freisetzende Verbindungen können darüber hinaus eingesetzt werden, wobei poröse Füllstoffe (insbesondere poröses SiO₂) besonders abrasionsstabile Dentalkomposite ergeben.

Auch übliche Vorbehandlungen wie die Hydrophobierung, Silanisierung und Säurebehandlung zur Konditionierung bzw. Reinigung der Oberflächen können angewendet werden.

Die Erfindung betrifft auch eine Dentalsystem, umfassend eine Dentalzusammensetzung, wie oben beschrieben und ein LED-gespeistes Lichtgerät mit einem Emissionsspektrum im Bereich von 420 - 520 nm.

Vorzugsweise liegt dabei das Emissionsmaximum bei 468 ± 10 nm, und im Wellenlängenbereich von 420 - 520 nm wird bei idealer Gauß'scher Verteilung mehr als 90 % der Energie emittiert.

Das LED-Gerät des Dentalsystems kann nach dem Laserprinzip betrieben werden.

Die Erfindung wird anhand der nachfolgenden Beispiele näher erläutert.

### Beispiel A

In einer Mischung aus 70 Teilen Bis-GMA (Bisphenol-A-Glycidyl-Dimethacrylat) und 30 Teilen TEGDMA (Triethylenglycoldimethacrylat) werden 6 Gewichts-% hochdisperses Siliciumdioxid, 70 Gewichts-% eines feinstgemahlenen Dentalglasfüllers (d₅₀ = 0,7 µm) eingearbeitet und mit 0,045 Gewichts-% Campherchinon und 1) 0,08 Gewichts-%, und 2) 0,4 Gewichts-% eines tert. Amins aktiviert.

### Beispiel B

In einer Mischung aus 70 Teilen Bis-GMA und 30 Teilen TEGDMA werden 6 Gewichts-% hochdisperses Siliciumdioxid, 70 Gewichts-% eines feinstgemahlenen Glasfüllers (d₅₀ = 0,7 µm) eingearbeitet und mit 0,09 Gewichts-% Campherchinon und 1) 0,08 Gewichts-% und 2) 0,4 Gewichts-% eines tert. Amins aktiviert.

### Beispiel C

In einer Mischung aus 70 Teilen Bis-GMA und 30 Teilen TEGDMA werden 6 Gewichts-% hochdisperses Siliciumdioxid, 70 Gewichts-% eines feinstgemahlenen Glasfüllers (d₅₀ = 0,7 µm) eingearbeitet und mit 0,04 Gewichts-% Campherchinon, 0,4 Gewichts-% eines tert. Amins und 1) 0,2 Gewichts-% und 2) 0,8 Gewichts-% 1-Phenyl-1,2-propandion aktiviert.

### Beispiel D

In einer Mischung aus 60 Teilen Bis-GMA, 30 Teilen eines Tetraacrylates und 10 Teilen TEGDMA werden 6 Gewichts-% hochdisperses Siliciumdioxid, 70 Gewichts-% eines feinstgemahlenen Glasfüllers (d₅₀ = 0,7 µm) eingearbeitet und mit 0,08 Gewichts-% Campherchinon und 1) 0,08 Gewichts-% und 2) 0,4 Gewichts-% eines tert. Amins gelöst.

### Ergebnisse

Als Maß für die Reaktionsgeschwindigkeit wurde die Härtebestimmung (Vickers-Härte) an der Unterseite eines 2 mm dicken Prüfkörper herangezogen. Als Referenz diente ein Universalkomposit der Farbe I (= Inzisal; unpigmentiertes Charisma von Heraeus Kulzer). Die Aushärtung der Referenzprobe erfolgte mit einer Halogenlampe (z. B. Translux CL, Heraeus Kulzer). Die aufgeführten Beispiele A, B und C wurden mit einer LED-Lampe ausgehärtet.

Folgende Schlussfolgerungen können aus den Ergebnissen der Härte-Messungen gezogen werden:
1. Die Härtung von experimentellen Mischungen durch eine LED-Lichtquelle (Beispiele A1 bis C2) führt bei gleicher Belichtungszeit zu vergleichbaren oder höheren Härte-Werten im Vergleich zu einer mit Halogen-Licht gehärteten Referens (Charisma Farbe I).
2. Bei gleicher Belichtungszeit mit einer LED-Lichtquelle führt die alleinige Erhöhung des Gehalts an tert. Amin im System Campherchinon / tert. Amin nicht zur Erhöhung der Härte-Werte (Beispiele A1 und A2).
3. Die Verdoppelung des Gehalts an Campherchinon im System Campherchinon / tert. Amin allein führt bei gleicher Belichtungszeit mit einer LED-Lichtquelle nicht zur Erhöhung der Härte-Werte (Beispiele B1 und B2).
4. Nur der Zusatz von 1-Phenyl-1,2-propandion zu dem Initiatorsystem Campherchinon / tert. Amin (C1 und C2) führt im Vergleich zu Proben ohne 1-Phenyl-1,2-propandion (A1 bis B2) zu einer wesentlichen Erhöhung der Härte der Proben bei gleicher Belichtungszeit mit einer LED-Lichtquelle.
5. Insbesondere erreichen die Mischungen mit Zusatz von 1-Pheyl-1,2-propandion (C1 und C2) bereits nach 10 Sek. Härtung mit einer LED-Lichtquelle im Rahmen der Messgenauigkeit gleiche oder höhere Härte-Werte als Proben ohne 1-Phenyl-1,2-propandion (A1 bis B2) nach 20 Sek. Härtung mit einer LED-Lichtquelle.

In der folgenden Tabelle sind die Ergebnisse der Härtemessungen im Detail zusammengestellt.

| **Ansatz** | **Lampe** | **Bestrahlungszeit** [Sekunden] | **Härte Unterseite** [MPA] Streuung der Werte +/- 5% |
|---|---|---|---|
| Referenz | Halogenlampe | 10 | 360 |
| | | 15 | 431 |
| | | **20** | **442** |
| A1 | LED-Lampe | 10 | 424 |
| | | 15 | 450 |
| | | **20** | **457** |
| A2 | LED-Lampe | 10 | 429 |
| | | 15 | 468 |
| | | **20** | **474** |
| B1 | LED-Lampe | 10 | 427 |
| | | 15 | 437 |
| | | **20** | **471** |
| B2 | LED-Lampe | 10 | 424 |
| | | 15 | 444 |
| | | **20** | **467** |
| C1 | LED-Lampe | **10** | **482** |
| | | 15 | 497 |
| | | 20 | 523 |
| C2 | LED-Lampe | **10** | **470** |
| | | 15 | 506 |
| | | 20 | 531 |

## Patentansprüche

1. Verfahren zum Aushärten einer (meth)acrylat-haltigen Dentalzusammensetzung, **dadurch gekennzeichnet, dass** als Photoinitiatorsystem
a) Campherchinon und/oder Phenanthrenchinon und
b) 1,-Phenyl-1,2 Propandion und/oder ein ähnliches Diketon und/oder Phosphinoxid,
c) kombiniert mit einem reduzierenden Amin verwendet wird
und mit blauem Licht aus einer LED-Quelle der Wellenlänge von 420 - 520 nm bestrahlt wird.

2. Verfahren nach Anspruch 1, **dadurch gekennzeichnet, dass** es sich beim reduzierenden Amin um eine Verbindung aus der Gruppe der tertiären aromatischen Amine handelt.

3. Verfahren nach Anspruch 1 oder 2, **dadurch gekennzeichnet, dass** es sich bei den (Meth)acrylaten um Urethandi(meth)acrylat, Bisphenol-A-Propyloxy-Di(meth)acrylat, Bisphenol-A-Glycidyl-Di(meth)acrylat, Esther-Urethan-(meth)acrylsäure-derivate eines Tricyclo-[5.2.1.0]-Decanderivates sowie cyclische Carbonatacrylat/methacrylat-Verbindungen handelt.

4. Verfahren nach einem der Ansprüche 1 bis 3, **dadurch gekennzeichnet, dass** mindestens ein Reaktivverdünner zugegen ist.

5. Verfahren nach Anspruch 4, **dadurch gekennzeichnet, dass** es sich beim Reaktivverdünner um einen Reaktivverdünner aus der Gruppe der aliphatischen Di(meth)acrylate und/oder Ethylenglycol-di(meth)acrylate und Polyethylenglycoldi(meth)-acrylate und/oder Hydroxyethyl(meth)acrylate handelt.

6. Verfahren nach einem der Ansprüche 1 bis 5, **dadurch gekennzeichnet, dass** mindestens ein Mehrfachvernetzer zugegen ist.

7. Verfahren nach Anspruch 6, **dadurch gekennzeichnet, dass** es sich beim Mehrfachvernetzer um einen Mehrfachvernetzer aus der Gruppe der (Meth)acrylate handelt.

8. Verfahren nach Anspruch 7, **dadurch gekennzeichnet, dass** es sich beim Mehrfachvernetzer um alkoxyliertes Pentaerythritol-Tetra(meth)acrylat oder 1,1,1-Trimethylolpropantri(meth)acrylat handelt.

## Claims

1. Process for curing a (meth)acrylate-containing dental composition, **characterized in that**
a) camphorquinone and/or phenanthrenequinone and
b) 1-phenyl-1,2-propanedione and/or a similar diketone and/or phosphine oxide,
c) combined with a reducing amine, is used as a photoinitiator system and is exposed to blue light from an LED source of wavelength 420 - 520 nm.

2. Process according to Claim 1, **characterized in that** the reducing amine is a compound from the group consisting of the tertiary aromatic amines.

3. Process according to Claim 1 or 2, **characterized in that** the (meth)acrylates are urethane di(meth)acrylate, bisphenol-A-propoxy di(meth)acrylate, bisphenol-A-glycidyl di(meth)acrylate, ester-urethane-(meth)acrylic acid derivatives of a tricyclo[5.2.1.0]-decane derivative and cyclic carbonate-acrylate/methacrylate compounds.

4. Process according to any of Claims 1 to 3, **characterized in that** at least one reactive diluent is present.

5. Process according to Claim 4, **characterized in that** the reactive diluent is a reactive diluent from the group consisting of the aliphatic di(meth)acrylates and/or ethylene glycol di(meth)acrylates and polyethylene glycol di(meth)acrylates and/or hydroxyethyl (meth)acrylates.

6. Process according to any of Claims 1 to 5, **characterized in that** at least one multicrosslinking agent is present.

7. Process according to Claim 6, **characterized in that** the multicrosslinking agent is a multicrosslinking agent from the group consisting of the (meth)acrylates.

8. Process according to Claim 7, **characterized in that** the multicrosslinking agent is alkoxylated pentaerythrityl tetra(meth)acrylate or 1,1,1-trimethylolpropane tri(meth)acrylate.

## Revendications

1. Procédé pour durcir une composition dentaire contenant un (méth)acrylate, **caractérisé en ce que** l'on utilise en tant que système photo-inducteur
a) la camphoquinone et/ou la phénanthrène quinone et
b) la 1-phényl-1,2-propanedione et/ou une dicétone analogue et/ou un oxyde de phosphine,
c) en combinaison avec une amine réductrice,
et on irradie par la lumière bleue d'une source à LED de longueur d'onde 420 à 520 nm.

2. Procédé selon la revendication 1, **caractérisé en ce que** l'amine réductrice consiste en un composé du groupe des amines aromatiques tertiaires.

3. Procédé selon la revendication 1 ou 2, **caractérisé en ce que** les (méth)acrylates consistent en uréthane di(méth)acrylate, propyloxy-di-(méth)acrylate du bisphénol A, glycidyl-di(méth)acrylate du bisphénol A, dérivés ester-uréthane-(méth)acryliques d'un dérivé du tricyclo-[5.2.1.0]-décane ou carbonatacrylate/méthacrylates cycliques.

4. Procédé selon l'une des revendications 1 à 3, **caractérisé en ce que** l'on opère en présence d'au moins un diluant réactif.

5. Procédé selon la revendication 1, **caractérisé en ce que** le diluant réactif est choisi dans le groupe des di(méth)acrylates aliphatiques et/ou des di(méth)acrylates de l'éthylèneglycol et di(méth)acrylates de polyéthylèneglycols et/ou (méth)acrylates d'hydroxyéthyle.

6. Procédé selon l'une des revendications 1 à 5, **caractérisé en ce que** l'on opère en présence d'au moins un agent réticulant multiple.

7. Procédé selon la revendication 6, **caractérisé en ce que** l'agent réticulant multiple est choisi dans le groupe des (méth)acrylates.

8. Procédé selon la revendication 7, **caractérisé en ce que** l'agent réticulant multiple consiste en tétra(méth)acrylate du pentaérythritol ou tri(méth)acrylate du 1,1,1-triméthylolpropane alcoxylé.
